(19) Europäisches Patentamt
European Patent Office
Office européen des brevets

(11) Veröffentlichungsnummer: **0 094 552**
**B1**

(12) **EUROPÄISCHE PATENTSCHRIFT**

(45) Veröffentlichungstag der Patentschrift: **24.07.85**

(21) Anmeldenummer: **83104344.3**

(22) Anmeldetag: **03.05.83**

(51) Int. Cl.⁴: **C 07 C 87/30,** C 07 D 487/18, C 07 F 17/02, A 01 N 33/12, A 01 N 43/90, A 01 N 55/02

(54) **Jodpropargylammoniumsalze, Verfahren zu ihrer Herstellung und ihre Verwendung als Schädlingsbekämpfungsmittel.**

(30) Priorität: **14.05.82 DE 3218176**

(43) Veröffentlichungstag der Anmeldung: **23.11.83 Patentblatt 83/47**

(45) Bekanntmachung des Hinweises auf die Patenterteilung: **24.07.85 Patentblatt 85/30**

(84) Benannte Vertragsstaaten: **AT BE CH DE FR GB IT LI NL SE**

(56) Entgegenhaltungen: **CHEMICAL ABSTRACTS, Band 73, Nr. 17, 26. Oktober 1970, Seite 32, Nr. 84056r, Columbus, Ohio, USA I. HARUNA et al.: "Specific inhibitor for RNA replicase"**

(73) Patentinhaber: **BAYER AG, Konzernverwaltung RP Patentabteilung, D-5090 Leverkusen 1 Bayerwerk (DE)**

(72) Erfinder: **Schmitt, Hans-Georg, Dr., Gustav-Freytag-Strasse 2, D-5090 Leverkusen 1 (DE)**
Erfinder: **Reinecke, Paul, Dr., Lessingstrasse 11, D-5090 Leverkusen 3 (DE)**
Erfinder: **Paulus, Wilfried, Dr., Deswatinesstrasse 90, D-4150 Krefeld 1 (DE)**
Erfinder: **Genth, Hermann, Dr., Am Heckerhof 60, D-4150 Krefeld 1 (DE)**
Erfinder: **Radt, Walter, Dr., Bethelstrasse 12, D-4150 Krefeld 1 (DE)**

**EP 0 094 552 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue und bekannte Jodpropargylammoniumsalze, Verfahren zu ihrer Herstellung und ihre Verwendung als Schädlingsbekämpfungsmittel, insbesondere als Fungizide.

Es ist seit langem bekannt, dass N-sulfenylierte Dicarbonsäureimide eine fungizide Wirksamkeit besitzen (vgl. DBP Nr. 1193498). Weiterhin sind Bis-(halogenalkinyloxymethyl)ammoniumsalze als Korrosionsinhibitoren bzw. als Mikrobizide zur Wasserbehandlung beschrieben (vgl. US-Patentschrift Nr. 4206233). Über ihren Einsatz zur Bekämpfung von pilzlichen Krankheiten ist nichts gesagt.

Weiterhin sind Halogenpropargylaminderivate wie z. B. das 3-Jodpropargyltrimethylammoniumbromid, bekannt. Über eine Wirkung als Fungizide ist nichts bekannt (vgl. „Ann. N.Y. Acad. Sci." [1970], 173 [Art. 1], 404-416; C.A. 73: 840 56r).

Es wurden neue Jodpropargylammoniumsalze der Formel IA

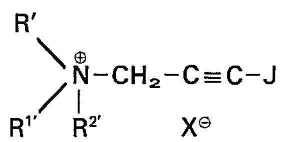 (IA)

gefunden, in welcher

R' und R1' gleich oder verschieden sind und für einen aliphatischen Rest stehen,

R2' für einen aliphatischen Rest, für einen ggf. ein- oder mehrfach, gleich oder verschieden substituierten Aralkylrest oder für einen Ferrocenmethylrest steht, oder

R', R1' und R2' gemeinsam mit dem Stickstoffatom einen Ring bilden, der durch weitere Stickstoffatome unterbrochen sein kann, und

X'⊖ für ein Anion steht, ausgenommen die Verbindung in der R', R1' und R2' für je einen Methylrest und X'⊖ für ein Bromanion stehen.

Man erhält die Jodpropargylammoniumsalze der Formel IA

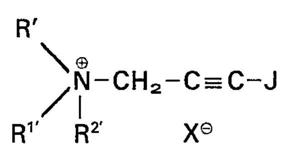 (IA)

in welcher

R' und R1' gleich oder verschieden sind und für einen aliphatischen Rest stehen,

R2' für einen aliphatischen Rest, für einen ggf. ein- oder mehrfach, gleich oder verschieden substituierten Aralkylrest oder für einen Ferrocenmethylrest steht, oder

R', R1' und R2' gemeinsam mit dem Stickstoffatom einen Ring bilden, der durch weitere Stickstoffatome unterbrochen sein kann und

X'⊖ für ein Anion steht, ausgenommen die Verbindung in der R', R1' und R2' für je einen Methylrest und X'⊖ für ein Bromanion stehen,

dadurch, dass man

a) tertiäre Amine der Formel II

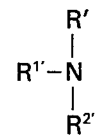 (II)

in welcher

R', R1' und R2' die angegebene Bedeutung haben, mit einer Jodpropargylverbindung der Formel III

$$J-C\equiv C-CH_2-X^1 \qquad (III)$$

in welcher

$X^1$ für Halogen oder einen Sulfonatrest steht, ggf. in Gegenwart eines Verdünngsmittels bei Temperaturen zwischen 0 und 110° C umsetzt oder

b) tertiäre Jodpropargylamine der Formel IV

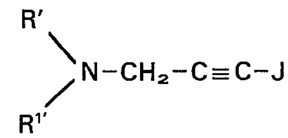 (IV)

in welcher

R' und R1' die oben angegebenen Bedeutungen haben, mit Verbindungen der Formel V

$$R^{2'}X^2 \qquad (V)$$

in welcher

R2' die oben angegebene Bedeutung hat und

$X^2$ für Halogen, Alkylsulfat oder für den Alkyl- bzw. Arylsulfonatrest steht, ggf. in Gegenwart eines Verdünnungsmittels bei Temperaturen zwischen 0 und 110° C umsetzt oder

c) die unter anderem unter a bzw. b erhaltenen erfindungsgemässen Jodpropargylammoniumhalogenide der Formel VI

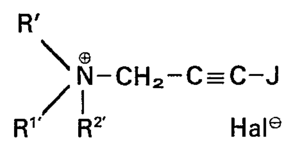 (VI)

in welcher

R', R1' und R2' die oben angegebenen Bedeutungen haben, und

Hal⊖ für ein Halogenanion steht, mit Verbindungen der Formel VII

$$B-Y \qquad (VII)$$

in welcher

Y für einen anionischen Rest steht mit der vorzugsweisen Bedeutung von X⊖ ausser Halogen und B für ein Alkali- oder Erdalkalikation steht, umsetzt.

Ausserdem wurde gefunden, dass die Jodpropargylammoniumsalze der Formel I

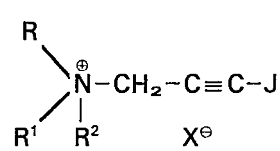 (I)

in welcher

R und $R^1$ gleich oder verschieden sind und für einen aliphatischen Rest stehen,

$R^2$ für einen aliphatischen Rest, für einen ggf. ein- oder mehrfach, gleich oder verschieden substituierten Aralkylrest oder für einen Ferrocenmethylrest steht oder

R, $R^1$ und $R^2$ gemeinsam mit dem Stickstoffatom einen Ring bilden, der durch weitere Stickstoffatome unterbrochen sein kann und

$X^\ominus$ für ein Anion steht,

starke mikrobizide Eigenschaften aufweisen. Überraschenderweise zeigen dabei die erfindungsgemässen Verbindungen eine erheblich höhere Wirkung als die aus dem Stand der Technik bekannten N-sulfenylierten Dicarbonsäureimide, wie das N-Trichlormethylthiotetrahydrophthalimid, welches eine wirkungsmässig naheliegende Verbindung ist.

Von den neuen Jodpropargylammoniumsalzen der Formel IA sind bevorzugt diejenigen, bei denen

R' und $R^{1'}$ gleich oder verschieden sind und für einen geradkettigen oder verzweigten Alkylrest stehen,

$R^{2'}$ für einen geradkettingen oder verzweigten Alkylrest, für einen ggf. ein bis fünffach im Phenylring substituierten Benzyl- oder Phenylethylrest oder für den Ferrocenmethylrest steht oder

R', $R^{1'}$ und $R^{2'}$ gemeinsam mit dem Stickstoffatom einen Urotropinrest bilden und

$X'^\ominus$ für ein Halogen-, Phosphat-, Acetat-, Benzoat-, Citrat-, Tartrat-, Alkyl- bzw. Arylsulfonat-, Alkylsulfat, Benzisothiazolinon- oder Saccharin-Anion steht, ausgenommen die Verbindung in der R', $R^{1'}$ und $R^{2'}$ für je einen Methylrest und $X'^\ominus$ für ein Bromanion stehen.

Besonders bevorzugt sind Jodpropargylammoniumsalze der Formel IA, in welcher

R' und $R^{1'}$ gleich oder verschieden sind und für geradkettiges oder verzweigtes Alkyl mit 1 bis 20 Kohlenstoffatomen je Alkylrest stehen,

$R^{2'}$ für geradkettiges oder verzweigtes Alkyl mit 4 bis 20 Kohlenstoffatomen für Ferrocenmethyl, für Benzyl oder Phenethyl steht, wobei jeweils der Phenylring ein- bis fünffach, gleich oder verschieden durch Alkyl und Halogen substituiert sein kann oder

R', $R^{1'}$ und $R^{2'}$ gemeinsam mit dem Stickstoffatom den Urotropinrest bilden, und

$X'^\ominus$ für ein Chlor-, Phosphat-, Acetat, Benzoat-, Citrat-, Tartrat-, Benzisothiazolinon- oder Saccharin-Anion, für Alkylsulfonat mit 1 bis 3 Kohlenstoffatomen im Alkylteil, vorzugsweise Methyl- und Ethylsulfonat, für Arylsulfonat, worin der Arylrest ggf. ein- oder zweifach durch Alkyl mit 1 bis 3 Kohlenstoffatomen substituiert sein kann, vorzugsweise Phenyl- und Toluolsulfonat, oder für Alkylsulfat mit 1 bis 3 Kohlenstoffatomen je Alkylrest, vor allem Methyl- und Ethylsulfatrest, steht.

Neben den in den Herstellungsbeispielen genannten Verbindungen der Formel IA seien noch im einzelnen die folgenden Verbindungen genannt:

(1-Jodpropargyl)-di-methyl-n-butylammoniumchlorid

(1-Jodpropargyl)-di-ethyl-n-butylammoniumchlorid

(1-Jodpropargyl)-di-n-propyl-n-butylammoniumchlorid

(1-Jodpropargyl)-di-isopropyl-n-butylammoniumchlorid

(1-Jodpropargyl)-di-isobutyl-n-butylammoniumchlorid

(1-Jodpropargyl)-di-sec.-butyl-n-butylammoniumchlorid

(1-Jodpropargyl)-di-pentyl-n-butylammoniumchlorid

(1-Jodpropargyl)-di-hexyl-n-butylammoniumchlorid

(1-Jodpropargyl)-di-methylheptyl-n-butylammoniumchlorid

(1-Jodpropargyl)-di-octyl-n-butylammoniumchlorid

(1-Jodpropargyl)-di-nonyl-n-butylammoniumchlorid

(1-Jodpropargyl)-di-decyl-n-butylammoniumchlorid

(1-Jodpropargyl)-di-dodecyl-n-butylammoniumchlorid

(1-Jodpropargyl)-di-hexylmethylammoniumchlorid

(1-Jodpropargyl)-di-pentylmethylammoniumchlorid

(1-Jodpropargyl)-di-n-butylmethylammoniumchlorid

(1-Jodpropargyl)-di-sec.-butylmethylammoniumchlorid

(1-Jodpropargyl)-di-isobutylmethylammoniumchlorid

(1-Jodpropargyl)-di-pentylmethylammoniumchlorid

(1-Jodpropargyl)-di-hexylmethylammoniumchlorid

(1-Jodpropargyl)-di-heptylmethylammoniumchlorid

(1-Jodpropargyl)-di-octylmethylammoniumchlorid

(1-Jodpropargyl)-di-nonylmethylammoniumchlorid

(1-Jodpropargyl)-di-decylmethylammoniumchlorid

(1-Jodpropargyl)-di-dodecylmethylammoniumchlorid

(1-Jodpropargyl)-di-pentylethylammoniumchlorid

(1-Jodpropargyl)-di-hexylethylammoniumchlorid

(1-Jodpropargyl)-di-heptylethylammoniumchlorid

(1-Jodpropargyl)-di-octylethylammoniumchlorid

(1-Jodpropargyl)-di-nonylethylammoniumchlorid

(1-Jodpropargyl)-di-decylethylammoniumchlorid

(1-Jodpropargyl)-di-dodecylethylammoniumchlorid

(1-Jodpropargyl)-di-methylbenzylammoniumchlorid

(1-Jodpropargyl)-di-ethylbenzylammoniumchlorid

(1-Jodpropargyl)-di-n-propylbenzylammoniumchlorid
(1-Jodpropargyl)-di-isopropylbenzylammoniumchlorid
(1-Jodpropargyl)-di-n-butylbenzylammoniumchlorid
(1-Jodpropargyl)-di-isobutylbenzylammoniumchlorid
(1-Jodpropargyl)-di-sec.-butylbenzylammoniumchlorid
(1-Jodpropargyl)-di-pentylbenzylammoniumchlorid
(1-Jodpropargyl)-di-hexylbenzylammoniumchlorid
(1-Jodpropargyl)-di-heptylbenzylammoniumchlorid
(1-Jodpropargyl)-di-octylbenzylammoniumchlorid
(1-Jodpropargyl)-di-nonylbenzylammoniumchlorid
(1-Jodpropargyl)-di-decylbenzylammoniumchlorid
(1-Jodpropargyl)-di-dodecylbenzylammoniumchlorid
(1-Jodpropargyl)-di-methylphenethylammoniumchlorid
(1-Jodpropargyl)-di-ethylphenethylammoniumchlorid
(1-Jodpropargyl)-di-n-propylphenethylammoniumchlorid
(1-Jodpropargyl)-di-isopropylphenethylammoniumchlorid
(1-Jodpropargyl)-di-n-butylphenethylammoniumchlorid
(1-Jodpropargyl)-di-sec.-butylphenethylammoniumchlorid
(1-Jodpropargyl)-di-isobutylphenethylammoniumchlorid
(1-Jodpropargyl)-di-pentylphenethylammoniumchlorid
(1-Jodpropargyl)-di-hexylphenethylammoniumchlorid
(1-Jodpropargyl)-di-heptylphenethylammoniumchlorid
(1-Jodpropargyl)-di-octylphenethylammoniumchlorid
(1-Jodpropargyl)-di-nonylphenethylammoniumchlorid
(1-Jodpropargyl)-di-decylphenethylammoniumchlorid
(1-Jodpropargyl)-di-dodecylphenethylammoniumchlorid
(1-Jodpropargyl)-tripentylammoniumchlorid
(1-Jodpropargyl)-triheptylammoniumchlorid
(1-Jodpropargyl)-trinonylammoniumchlorid
(1-Jodpropargyl)-di-methyl-(2-chlorphenethyl)ammoniumchlorid
(1-Jodpropargyl)-di-ethyl-(2-chlorphenethyl)-ammoniumchlorid
(1-Jodpropargyl)-di-propyl-(2-chlorphenethyl)ammoniumchlorid
(1-Jodpropargyl)-di-butyl-(2-chlorphenethyl)-ammoniumchlorid
(1-Jodpropargyl)-di-pentyl-(2-chlorphenethyl)ammoniumchlorid
(1-Jodpropargyl)-di-hexyl-(2-chlorphenethyl)ammoniumchlorid
(1-Jodpropargyl)-di-heptyl-(2-chlorphenethyl)ammoniumchlorid
(1-Jodpropargyl)-di-octyl-(2-chlorphenethyl)ammoniumchlorid
(1-Jodpropargyl)-di-nonyl-(2-chlorphenethyl)ammoniumchlorid
(1-Jodpropargyl)-di-methyl-(4-chlorphenethyl)ammoniumchlorid
(1-Jodpropargyl)-di-ethyl-(4-chlorphenethyl)ammoniumchlorid
(1-Jodpropargyl)-di-propyl-(4-chlorphenethyl)ammoniumchlorid
(1-Jodpropargyl)-di-butyl-(4-chlorphenethyl)ammoniumchlorid
(1-Jodpropargyl)-di-pentyl-(4-chlorphenethyl)ammoniumchlorid
(1-Jodpropargyl)-di-hexyl-(4-chlorphenethyl)ammoniumchlorid
(1-Jodpropargyl)-di-heptyl-(4-chlorphenethyl)ammoniumchlorid
(1-Jodpropargyl)-di-octyl-(4-chlorphenethyl)ammoniumchlorid
(1-Jodpropargyl)-di-nonyl-(4-chlorphenethyl)ammoniumchlorid
(1-Jodpropargyl)-di-decyl-(4-chlorphenethyl)ammoniumchlorid
(1-Jodpropargyl)-di-dodecyl-(4-chlorphenethyl)ammoniumchlorid
(1-Jodpropargyl)-di-methyldodecylammoniumchlorid
(1-Jodpropargyl)-di-ethyldodecylammoniumchlorid
(1-Jodpropargyl)-di-propyldodecylammoniumchlorid
(1-Jodpropargyl)-di-butyldodecylammoniumchlorid
(1-Jodpropargyl)-di-pentyldodecylammoniumchlorid
(1-Jodpropargyl)-di-hexyldodecylammoniumchlorid
(1-Jodpropargyl)-di-heptyldodecylammoniumchlorid
(1-Jodpropargyl)-di-octyldodecylammoniumchlorid
(1-Jodpropargyl)-di-nonyldodecylammoniumchlorid
(1-Jodpropargyl)-di-decyldodecylammoniumchlorid
(1-Jodpropargyl)-di-methyl-(2,4-dichlorbenzyl)ammoniumchlorid
(1-Jodpropargyl)-di-ethyl-(2,4-dichlorbenzyl)ammoniumchlorid
(1-Jodpropargyl)-di-propyl-(2,4-dichlorbenzyl)ammoniumchlorid
(1-Jodpropargyl)-di-butyl-(2,4-dichlorbenzyl)ammoniumchlorid
(1-Jodpropargyl)-di-pentyl-(2,4-dichlorbenzyl)ammoniumchlorid
(1-Jodpropargyl)-di-hexyl-(2,4-dichlorbenzyl)ammoniumchlorid
(1-Jodpropargyl)-di-heptyl-(2,4-dichlorbenzyl)ammoniumchlorid

(1-Jodpropargyl)-di-methyl-(2,4,6-trichlorbenzyl)ammoniumchlorid
(1-Jodpropargyl)-di-ethyl-(2,4,6-trichlorbenzyl)ammoniumchlorid
(1-Jodpropargyl)-di-propyl-(2,4,6-trichlorbenzyl)ammoniumchlorid
(1-Jodpropargyl)-di-butyl-(2,4,6-trichlorbenzyl)ammoniumchlorid
(1-Jodpropargyl)-di-pentyl-(2,4,6-trichlorbenzyl)ammoniumchlorid
(1-Jodpropargyl)-di-hexyl-(2,4,6-trichlorbenzyl)ammoniumchlorid
(1-Jodpropargyl)-di-heptyl-(2,4,6-trichlorbenzyl)ammoniumchlorid
(1-Jodpropargyl)-di-methyl-(2-methylbenzyl)ammoniumchlorid
(1-Jodpropargyl)-di-ethyl-(2-methylbenzyl)ammoniumchlorid
(1-Jodpropargyl)-di-propyl-(2-methylbenzyl)ammoniumchlorid
(1-Jodpropargyl)-di-butyl-(2-methylbenzyl)ammoniumchlorid
(1-Jodpropargyl)-di-pentyl-(2-methylbenzyl)ammoniumchlorid
(1-Jodpropargyl)-di-hexyl-(2-methylbenzyl)ammoniumchlorid
(1-Jodpropargyl)-di-heptyl-(2-methylbenzyl)ammoniumchlorid
(1-Jodpropargyl)-di-methyl-(2,6-dimethylbenzyl)ammoniumchlorid
(1-Jodpropargyl)-di-ethyl-(2,6-dimethylbenzyl)ammoniumchlorid
(1-Jodpropargyl)-di-propyl-(2,6-dimethylbenzyl)ammoniumchlorid
(1-Jodpropargyl)-di-butyl-(2,6-dimethylbenzyl)ammoniumchlorid
(1-Jodpropargyl)-di-pentyl-(2,6-dimethylbenzyl)ammoniumchlorid
(1-Jodpropargyl)-di-hexyl-(2,6-dimethylbenzyl)ammoniumchlorid
(1-Jodpropargyl)-di-heptyl-(2,6-dimethylbenzyl)ammoniumchlorid
(1-Jodpropargyl)-di-nonyl-(2,6-dimethylbenzyl)ammoniumchlorid

und die jeweils entsprechenden Alkylsulfat-, Phosphat-, Acetat-, Benzoat-, Citrat-, Tartrat-, Benzisothiazolinon- und Saccharinsalze, ferner die Alkyl- oder Arylsulfonatverbindungen.

Die einzelnen Verfahrensvarianten können wie folgt wiedergegeben werden:

Verwendet man beispielsweise bei Verfahrensvariante a Tripentylamin und Jodpropargylchlorid als Ausgangsstoffe, so kann der Reaktionsverlauf durch das folgende Formelschema beschrieben werden:

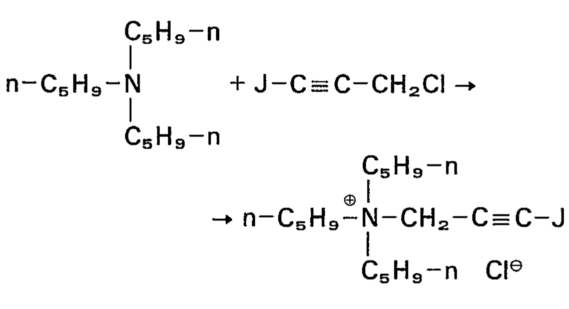

Verwendet man beispielsweise bei Verfahrensvariante b Dimethyl-3-jodpropargylamin und n-Butylchlorid als Ausgangsstoffe, so kann der Reaktionsverlauf durch das folgende Formelschema wiedergegeben werden:

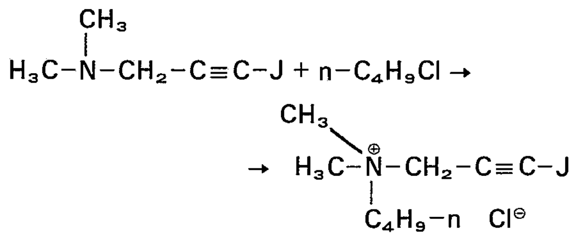

Verwendet man bei Verfahrensvariante c Trihexyl-(1-jodpropargyl)ammoniumchlorid und Natriumbenzoat als Ausgangsmaterialien, so kann der Reaktionsverlauf durch das folgenden Formelschema wiedergegeben werden:

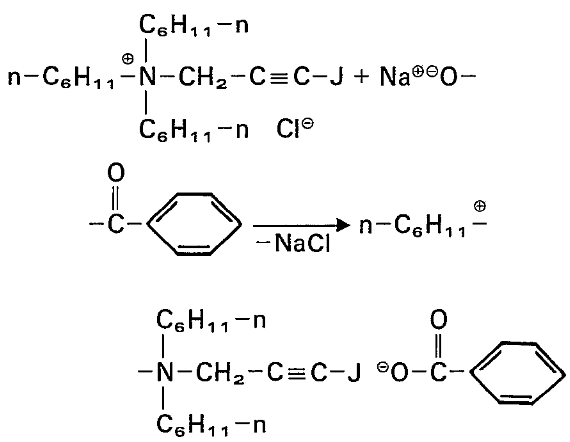


Die bei den oben beschriebenen Herstellungsverfahren eingesetzten tertiären Amine sind durch die Formel II allgemein definiert. Die Verbindungen sind bekannt und/oder können nach an sich bekannten Verfahren hergestellt werden (vgl. Houben-Weyl, Bd. 11/1, Thieme Verlag, Stuttgart, 1957). Die weiterhin als Ausgangsstoffe zu verwendenden Jodpropargylverbindungen der Formel III sind literaturbekannte Verbindungen oder nach bekannten Verfahren herstellbar (vgl. z. B. H.G. Viehe, „The Chemistry of Acetylenes", S. 689; M. Dekker, New York, 1969).

Die bei der Verfahrensvariante b einzusetzenden tertiären Jodpropargylamine sind durch die Formel IV beschrieben und grösstenteils bekannt oder können nach literaturbekannten Verfahren, z. B. durch Umsetzung von Propargylhalogeniden mit Dialkylaminen und anschliessender Jodierung, hergestellt werden (vgl. Jap. Patent Nr. 70.41008). Ebenso sind die weiterhin einzusetzenden Verbindungen der Formel V literaturbekannte Verbindungen.

Die bei der Verfahrensvariante c einzusetzenden Jodpropargylammoniumhalogenide der Formel VI sind neu und gehören zu dem Erfindungsgedanken. Sie können nach bekannten Verfahren aus tertiären Aminen und Jodpropargylhalogenid hergestellt werden.

Die weiterhin als Ausgangsverbindungen einzusetzenden Verbindungen der Formel VII sind

altbekannte und käuflich zu erwerbende Verbindungen, die nach bekannten Verfahren hergestellt werden können.

Die Verfahrensvariante a und b werden vorzugsweise unter Verwendung von Verdünnungsmitteln durchgeführt. Als Verdünnungsmittel kommen praktisch alle organischen Lösungsmittel mit einer gewissen Polarität in Frage. Hierzu gehören insbesondere halogenierte Kohlenwasserstoffe, wie Methylenchlorid, Ethylenchlorid, Chloroform, Tetrachlorkohlenstoff, Chlorbenzol und o-Dichlorbenzol. Weiterhin Ether, wie Diethyl- und Dibutylether, Glykoldimethylether, Tetrahydrofuran und Dioxan; Ketone wie Aceton, Methylethyl-, Methylisopropyl- und Methylisobutylketon; Ester, wie Essigsäuremethylester und -ethylester; Nitrile, wie z. B. Acetonitril und Propionitril; Aromaten, wie Benzol, Toluol und Xylol und auch Alkohole, wie z. B. Methanol, Ethanol, n- und iso-Propanol. Es können auch Gemische von Verdünnungsmitteln eingesetzt werden. Die Verfahrensvariante a kann auch in Wasser ausgeführt werden.

Die Reaktionstemperatur liegt bei den Verfahrensvarianten a und b zwischen 0 und 110° C, vorzugsweise zwischen 20 und 80° C. Die Verfahrensvarianten a und b werden vorzugsweise bei Normaldruck durchgeführt.

Der Austausch des Kations bei der Verfahrensvariante c erfolgt vorzugsweise in wässerigem Medium, bei Temperaturen wischen 20 und 100° C, vorzugsweise bei 20 bis 60° C und bei Normaldruck.

In einer bevorzugten Ausführungsform der erfindungsgemässen Verfahrensvariante a bzw. b legt man die Verbindungen der Formel II bzw. IV gelöst in einem der angeführten Verdünnungsmittel vor und gibt die Ausgangsverbindung der Formel III bzw. V zu. Die Reaktion ist schwach exotherm. Die Ausscheidung der erfindungsgemässen Verbindungen beginnt nach wenigen Minuten. Es wird aber meist über Nacht weitergerührt und am nächsten Tag das ausgefallene Produkt abgesaugt und getrocknet.

Bei der Durchführung der Verfahrensvariante c wird das erfindungsgemässe neue Ammoniumhalogenid der Formel VI in Wasser gelöst und mit einer wässerigen Lösung der Verbindungen der Formel VII zusammengegeben. Auch hier rührt man mehrere Stunden zur Vervollständigung der Reaktion, vorzugsweise bei Raumtemperatur, nach. Anschliessend wird das ausgeschiedene Produkt abgesaugt und getrocknet.

Der Anionenaustausch nach Verfahren c ist auch direkt möglich, d.h. die Jodpropargylammoniumhalogenide der Formel VI brauchen' zwischenzeitlich nicht isoliert zu werden, sondern können direkt in Lösung weiter mit Verbindungen der Formel VII umgesetzt werden.

Die bekannten Verbindung kann ebenfalls nach den genannten Verfahren hergestellt werden.

Die erfindungsgemäss verwendbaren Wirkstoffe der Formel I weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel und als mikrobizide Mittel zum Schutz technischer Materialien geeignet.

Zum Beispiel werden fungizide Mittel im Pflanzenschutz vor allem eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von *Pseudomonadaceae, Thizobiacecae, Enterobacteriaceae, Corynebacteriaceae* und *Streptomycetaceae* eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Besonders hervorzuheben ist die gute Wirksamkeit der erfindungsgemäss verwendbaren Verbindungen der Formel I im Pflanzenschutz gegen *Leptosphaeria nodorum* im Weizen. Ausserdem sollte die Wirkung gegen *Erysiphe, Puccinia, Cochliobolus sativus* und *Pyrenophora teres* an Getreide, die Wirkung gegen Apfelschorf und Oomyceten, die breite fungizide Wirkung im Agarplattentest gegen Reisindikationen und die bakterizide Wirkung dieser neuen Verbindungen erwähnt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, ggf. unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als

feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z. B. nichtionogene und anionische Emulgatoren, wie Polyoxyethylene-Fettsäure-Ester, Polyoxyethylen-Fettalkohol-Ether, z. B. Alkylarylpolyglykolether, Alkylsulfonate, Alkylsulfate, Arylsulfonate sowie Eiweisshydrolysate; als Dispergiermittel kommen in Frage: z. B. Lignin-Sulfit-Ablaugen und Methylzellulose.

Es können in den Formulierungen Haftmittel wie Carboxymethylcellulose, natürliche und synthetische pulvrige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäss verwendbaren Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Giessen, Tauchen, Spritzen, Sprühen, Vernebeln, Verdampfen, Injizieren, Verschlämmen, Verstreichen, Stäuben, Streuen, Trokkenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,001 bis 0,02%, am Wirkungsort erforderlich.

Die erfindungsgemässen Verbindungen eignen sich auch zum Schutz technischer Materialien, wie bereits beschrieben.

Technische Materialien im Rahmen der vorliegenden Erfindung sind Produkte, die selbst nicht in der Natur vorkommen, sondern aus natürlichen oder synthetischen Ausgangsmaterialien gefertigt werden. Die zu schützenden Produkte im Rahmen der vorliegenden Erfindung sind technische Materialien, die von Mikroorganismen befallen und/oder zersetzt werden können.

Technischen Materialien, die durch die erfindungsgemässen Substanzen vor einer mikrobiellen Veränderung und Zerstörung geschützt werden sollen, sind beispielsweise Klebstoffe, Leimen, Papiere und Kartone, Textilien, Leder, Holz, Anstrichmittel, Putze, Kühlschmiermittel, Dichtungsmassen und Kunststoffartikel, die von Mikroorganismen befallen oder zersetzt werden können. Im Rahmen der zu schützenden Materialien seien auch Teile von Produktionsanlagen, wie beispielsweise Kühlwasser- und Kühlschmiermittelkreisläufe, genannt, deren Funktionstüchtigkeit durch Mikroorganismen beeinträchtigt werden kann. Vorzugsweise können die erfindungsgemässen Wirkstoffe zum Schutz von Klebstoffen, Papier, Karton, Anstrichfilmen, Holz u. ä. verwendet werden.

Mikroorganismen, die einen Abbau oder eine Veränderung der technischen Materialien bewirken können, sind beispielsweise Bakterien, Pilze, Hefen, Algen u. Schleimorganismen. Vorzugsweise haben die erfindungsgemässen Substanzen eine starke und breite Wirkung gegen Pilze und Bakterien von der fungiziden Wirkung werden Schimmelpilze ebenso erfasst wie Holz-zerstörende und Holz-verfärbende Pilze.

Es seien beispielsweise Mikroorganismen der folgenden Gattungen genannt:

*Alternaria*, wie *Alternaria tenuis*,
*Aspergillus*, *Aspergillus niger*,
*Aureobasidium*, wie *Aureobasidium pullulans*,
*Chaetomium*, wie *Chaetomium globosum*,
*Coniophora*, wie *Coniophora cerebella*,
*Lentinus*, wie *Lentinus tigrinus*,
*Penicillium*, wie *Penicillium glaucum*,
*Polyporus*, wie *Polyporus versicolor*,
*Sclerophoma*, wie *Sclerophoma pityophila*,
*Trichoderma*, wie *Trichoderma viride*, bzw.
*Eschenichia*, wie *Eschenichia coli* oder
*Staphylococcus*, wie *Staphylococcus aureus*.

Je nach ihrem Anwendungsgebiet können die erfindungsgemässen Substanzen in die üblichen Formulierungen überführt werden, wie Lösungen, Emulsionen, Suspensionen, Pulver, Pasten und Granulate. Diese können in an sich bekannter Weise hergestellt werden, z. B. durch Vermischen der Wirkstoffe mit einem Streckmittel, das aus flüssigem Lösungsmittel und/oder festen Trägerstoffen, ggf. unter Verwendung von oberflächenaktiven Mitteln, wie Emulgatoren und/oder Dispergiermitteln, wobei beispielsweise im Falle der Benutzung von Verstreckmitteln, ggf. organische Solvenzien, als Hilfslösungsmittel verwendet werden können.

Organische Solvenzien für die Wirkstoffe können beispielsweise Alkohole, wie niedere Alkohole, vorzugsweise Ethanol oder Isopropanol, oder Benzylalkohol, Ketone wie Aceton oder Methylethylketon, flüssige Kohlenwasserstoffe, wie Benzinfraktionen, chlorierte Kohlenwasserstoffe, wie 1,2-Dichlorethan sein.

Die erfindungsgemässen mikrobiziden Mittel enthalten im allgemeinen 10 bis 100 Gew.-%, bevorzugt 50 bis 60 Gew.-%, der Jodpropargylammoniumsalze als Wirkstoff.

Die Anwendungskonzentration der erfindungsgemässen Substanzen richtet sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,01 bis 1 Gew.-%, bezogen auf das zu schützende Material.

Die erfindungsgemässen neuen Wirkstoffe können auch in Mischung mit anderen bekannten Wirkstoffen vorliegen. Beispielsweise seien die folgenden Wirkstoffe genannt: Benzimidazolylcarbamate, Trihalogenmethylthioverbindungen, wie N-Fluordichlormethylthiophthalimid und N,N-Dimethyl-N'-phenyl-N'-(fluordichlormethylthio)sulfamid, formaldehydabspaltende Verbindungen, wie Hemiformale, Phenolderivate, wie p-Chlor-m-kresol, 2-Phenylphenol, (2,2'-Dihydroxy-5,5'-dichlor)diphenylmethan, Dithiocarbamate, Thiazolylbenzimidazol, Isothiazolon- und Benzisothiazolonderivate, Tetrachlorisophthalsäurenitril, Mercaptobenzthiazol, Mercaptopyridin.

*Herstellungsbeispiele*

*Beispiel 1:*

(Variante a)

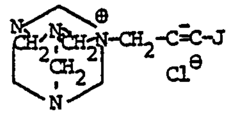

14 g (0,1 mol) Hexamethylentetramin werden in 800 ml Aceton gelöst und 20 g (0,1 mol) 3-Chlor-1-jodpropin zugegeben. Die Reaktion ist schwach exotherm, und nach ca. 15 min beginnt die Abscheidung des Quartärsalzes. Es wird über Nacht bei Raumtemperatur gerührt und anschliessend wird das hellbeige Produkt abgesaugt und getrocknet. Man erhält 28,2 g (84,7% der Theorie) an 1-N-(3-Jodpropargyl)hexamethylentetrammoniumchlorid vom Schmelzpunkt 193-195° C. Analog können die Verbindungen der Formel IA

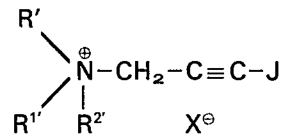 (IA)

enthalten werden:

| Bsp.-Nr. | R' | R1' | R2' | X'⊖ | Schmp. (° C) |
|---|---|---|---|---|---|
| 2 | $n-C_4H_9-$ | $n-C_4H_9-$ | $n-C_4H_9-$ | Cl | 167-169 |
| 3 | $C_6H_5-CH_2-$ | $CH_3$ | $CH_3$ | Cl | 160-162 |
| 4 | $n-C_8H_{17}-$ | $n-C_8H_{17}-$ | $n-C_8H_{17}-$ | Cl | Wachs |
| 5 | $n-C_6H_{13}-$ | $n-C_6H_{13}-$ | $n-C_6H_{13}-$ | Cl | Harz |
| 6 Gemisch | $\{n-C_{12}H_{25}-$<br>$n-C_{14}H_{29}-$ | $\{CH_3$<br>$CH_3$ | $\{CH_3$<br>$CH_3$ | Cl | 168-170 |
| 7 | 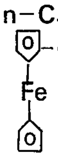 $-CH_2-$ | $CH_3$ | $CH_3$ | Cl | 300 |

*Beispiel 8:*

(Variante c)

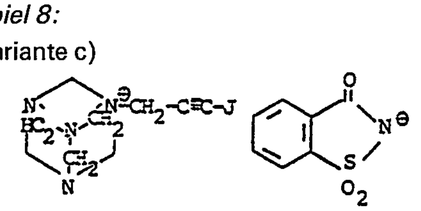

7,1 g (0,029 mol) Saccharin-Natriumdihydrat werden in 60 ml Wasser und 10 g (0,029 mol) 1-N-(3-Iodpropargyl)hexamethylentetrammoniumchlorid werden in 330 ml Wasser gelöst. Beide Lösungen werden vereinigt und bei Raumtemperatur gerührt. Nach ca. 30 min beginnt die Abscheidung des Produktes. Es wird über Nacht bei Raumtemperatur weitergerührt, dann abgesaugt und im Exsiccator getrocknet.

Man erhält 8,2 g (54% der Theorie) an 1-N-(3-Iodpropargyl)hexamethylentetrammoniumsaccharinat als weisses Pulver vom Schmelzpunkt 189° C.

*Beispiel 9:*

Ebenso wie in Beispiel 8 kann das 1-N-(3-Iodpropargyl)hexamethylentetrammonium(benzisothiazolin-3-on)at

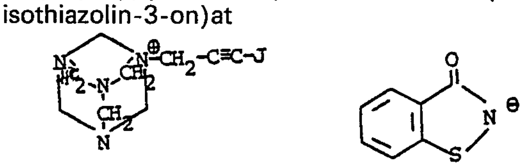

als hellbraunes Produkt in 48%iger Ausbeute vom Schmelzpunkt 103-105° C erhalten werden.

*Beispiel 10:*

Beispiel 10 kann ebenso wie in Beispiel 8 beschrieben hergestellt werden. Man erhält die Verbindung

Gemisch 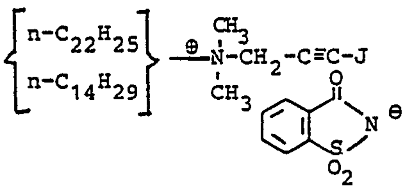

als weisses Pulver mit dem Schmelzpunkt 97-100° C.

*Verwendungsbeispiele*

In dem nachfolgenden Verwendungsbeispiel A wurde die nachstehend angegebene Vergleichssubstanz herangezogen:

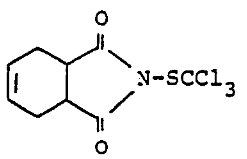

*Beispiel A:*

*Leptosphaeria nodorum*-Test (Weizen)/protektiv

Lösungsmittel: 100 Gew.-Teile Dimethylformamid

Emulgator: 0,25 Gew.-Teile Alkylarylpolyglykolether

Zur Herstellung einer zweckmässigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von *Leptosphaeria nodorum* besprüht. Die Pflanzen verbleiben 48 h bei 20° C und 100% relativer Luftfeuchtigkeit in einer Inkubationskabine.

Die Pflanzen werden in einem Gewächshaus bei einer Temperatur von ca. 15° C und einer relativen Luftfeuchtigkeit von ca. 80% aufgestellt.

10 Tage nach der Inokulation erfolgt die Auswertung.

Eine deutliche Überlegenheit in der Wirksamkeit gegenüber der aus dem Stand der Technik bekannten Verbindung zeigen bei diesem Test z. B. die Verbindungen gemäss folgenden Herstellungsbeispielen: 5, 4, 8 und 10.

*Beispiel B*

Wirkung gegen Pilze

In einem Agar, der aus Bierwürze und Pepton hergestellt wurde, wurden die erfindungsgemässen Verbindungen in abgestuften Konzentrationen zwischen 20 und 5000 mg/l Versuchsprobe eingearbeitet. Nach dem Erstarren des Agars erfolgte die Kontamination der so hergestellten Agarproben mit Reinkulturen verschiedener Testpilze, wie *Aspergillus niger, Chaetomium globosum* und *Penicillium glaucum*.

Nach zweiwöchiger Lagerung bei 28° C und 60 bis 70% relativer Luftfeuchtigkeit wurde ausgewertet. Es wird als minimale Hemmkonzentration (MHK) die geringste in einer Agarprobe enthaltene Konzentration der Substanz angegeben, bei der keinerlei Bewuchs durch die verwendete Art erfolgte.

In diesem Test zeigen Verbindungen gemäss Herstellungsbeispielen 6, 10, 4, 1, 7 und 3 eine sehr gute Wirkung.

*Beispiel C:*

Wirkung gegen Bakterien

Ein Agar, der als Nährmedium Bouillon enthält, wird mit den erfindungsgemässen Wirkstoffen in Konzentrationen von 20 bis 5000 ppm versetzt. Darauf infiziert man das Nährmedium jeweils mit *Escherichia coli* oder *Staphylococcus aureus* und hält das infizierte Medium 2 Wochen bei 28° C und 60 bis 70% relativer Luftfeuchtigkeit. Die MHK ist die niedrigste Konzentration an Wirkstoff, bei der keinerlei Bewuchs durch die verwendete Mikrobenart erfolgt.

In diesem test zeigen z. B. die Verbindungen gemäss Herstellungsbeispielen 6, 10, 4, 1, 7 und 3 gute Ergebnisse.

*Beispiel D:*

Wirkung gegen Schleimorganismen

Die Verbindungen werden in Konzentrationen von jeweils 0,1 bis 100 mg/l in Allens Nährlösung („Arch. Mikrobiol". *17*, 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1% Caprolactam enthält, in wenig Aceton gelöst, zur Anwendung gebracht. Kurz vorher wird die Nährlösung mit Schleimorganismen (ca. $10^6$ Keime/ml), die aus bei der Polyamidherstellung verwendeten Spinnwasser-Kreisläufen isoliert wurden, infiziert. Nährlösungen, die die minimale Hemmkonzentration (MHK) oder grössere Wirkstoffkonzentrationen aufweisen, sind nach 3wöchiger Kultur bei Raumtemperatur noch völlig klar, d. h. die in wirkstofffreien Nährlösungen nach 3 bis 4 Tagen bemerkbare starke Vermehrung der Mikroben und Schleimbildung unterbleibt.

In diesem Test zeigen die Verbindungen gemäss den Herstellungsbeispielen 6, 10, 4, 1, 7 und 3 gute Wirkungen.

**Patentansprüche**

1. Jodpropargylammoniumsalze der Formel (IA)

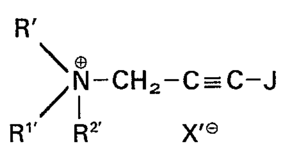 (IA)

in welcher

R′ und $R^{1'}$ gleich oder verschieden sind und für einen aliphatischen Rest stehen,

$R^{2'}$ für einen aliphatischen Rest, für einen ggf. ein- oder mehrfach, gleich oder verschieden substituierten Aralkylrest oder für einen Ferrocenmethylrest steht, oder

R′, $R^{1'}$ und $R^{2'}$ gemeinsam mit dem Stickstoffatom einen Ring bilden, der durch weitere Stickstoffatome unterbrochen sein kann, und

$X'^{\ominus}$ für ein Anion steht, ausgenommen die Verbindung, in der R′, $R^{1'}$ und $R^{2'}$ für je einen Methylrest und $X'^{\ominus}$ für ein Bromanion stehen.

2. Jodpropargylammoniumsalze der Formel (IA) nach Anspruch 1, worin

R′ und $R^{1'}$ gleich oder verschieden sind und für einen geradkettigen oder verzweigten Alkylrest stehen,

$R^{2'}$ für einen geradkettigen oder verzweigten Alkylrest, für einen ggf. ein- bis fünffach im Phenylring substituierten Benzyl- oder Phenethylrest oder für den Ferrocenmethylrest steht, oder

R′, $R^{1'}$ und $R^{2'}$ gemeinsam mit dem Stickstoffatom einen Urotropinrest bilden, und

$X'^{\ominus}$ für ein Halogen-, Phosphat-, Acetat-, Benzoat-, Citrat-, Tartrat-, Sulfonat-, Sulfat-, Benzisothiazolinon- oder Saccharin-Anion steht, ausgenommen die Verbindung, in der R′, $R^{1'}$ und $R^{2'}$ für je einen Methylrest und $X'^{\ominus}$ für ein Bromanion stehen.

3. Jodpropargylammoniumsalze der Formel (IA) nach Anspruch 1, worin

R′ und $R^{1'}$ gleich oder verschieden sind und für geradkettiges oder verzweigtes Alkyl mit 1 bis 20 Kohlenstoffatomen je Alkylrest stehen,

$R^{2'}$ für geradkettiges oder verzweigtes Alkyl mit 4 bis 20 Kohlenstoffatomen, für Benzyl oder Phenethyl steht, wobei jeweils der Phenylring ein- bis fünffach, gleich oder verschieden durch Alkyl und Halogen substituiert sein kann oder für Ferrocenmethyl steht, oder

R′, $R^{1'}$ und $R^{2'}$ gemeinsam mit dem Stickstoffatom den Urotropinrest bilden, und

$X'^{\ominus}$ für ein Chlor-, Phosphat-, Acetat-, Benzoat-, Citrat-, Tartrat-, Benzisothiazolinon- oder Saccharin-Anion, für einen Alkylsulfonatrest mit 1 bis 3 Kohlenstoffatomen im Alkylteil, für einen Arylsulfonatrest, der ggf. ein- oder zweifach durch Alkyl mit 1 bis 3 Kohlenstoffatomen substituiert sein kann oder für einen Alkylsulfatrest mit 1 bis 3 Kohlenstoffatomen im Alkylrest steht.

4. Verfahren zur Herstellung von Jodpropargylammoniumsalzen der Formel (IA)

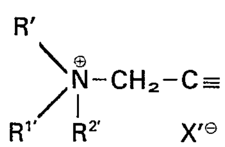 (IA)

in welcher

R′ und $R^{1'}$ gleich oder verschieden sind und für einen aliphatischen Rest stehen,

$R^{2'}$ für einen aliphatischen Rest, für einen ggf. ein- oder mehrfach, gleich oder verschieden substituierten Aralkylrest oder für einen Ferrocenmethylrest steht, oder

R′, $R^{1'}$ und $R^{2'}$ gemeinsam mit dem Stickstoffatom einen Ring bilden, der durch weitere Stickstoffatome unterbrochen sein kann, und

$X'^{\ominus}$ für ein Anion steht, ausgenommen die Verbindung, in welcher R′, $R^{1'}$ und $R^{2'}$ für je einen Methylrest und $X'^{\ominus}$ für ein Bromanion stehen, dadurch gekennzeichnet, dass man

a) tertiäre Amine der Formel (II)

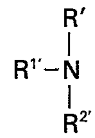 (II)

in welcher

R′, $R^{1'}$ und $R^{2'}$ die angegebene Bedeutung haben, mit einer Jodpropargylverbindung der Formel (III)

$$J-C\equiv C-CH_2-X^1 \quad (III)$$

in welcher

$X^1$ für Halogen oder einen Sulfonatrest steht, ggf. in Gegenwart eines Verdünnungsmittels bei Temperaturen zwischen 0 und 110° C umsetzt, oder

b) tertiäre Jodpropargylamine der Formel (IV)

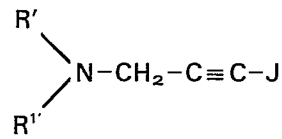 (IV)

in welcher

R′ und $R^{1'}$ die oben angegebenen Bedeutungen haben, mit Verbindungen der Formel (V)

$$R^{2'}X^2 \quad (V)$$

in welcher

$R^{2'}$ die oben angegebene Bedeutung hat, und

$X^{2'}$ für Halogen, Alkylsulfat oder für den Alkyl- oder Arylsulfonatrest steht, ggf. in Gegenwart eines Verdünnungsmittels bei Temperaturen zwischen 0 und 110° C umsetzt, oder

c) die erhaltenen erfindungsgemässen Jodpropargylammoniumhalogenide der Formel (VI)

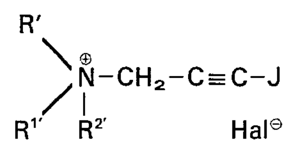 (VI)

in welcher

R′, $R^{1'}$ und $R^{2'}$ die oben angegebenen Bedeutungen haben, und

$Hal^{\ominus}$ für ein Halogenanion steht, mit Verbindungen der Formel (VII)

$$B-Y \quad (VII)$$

in welcher

Y für einen anionischen Rest steht mit der vorzugsweisen Bedeutung von $X^{\ominus}$ ausser Halogen, und

B für ein Alkali- oder Erdalkalikation steht, umsetzt.

5. Schädlingsbekämpfungsmittel, gekennzeichnet durch einen Gehalt an mindestens einem Jodpropargylammoniumsalz der Formel (I)

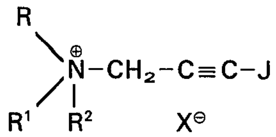
(I)

in welcher

R und $R^1$ gleich oder verschieden sind für einen aliphatischen Rest stehen,

$R^2$ für einen aliphatischen Rest, für einen ggf. ein- oder mehrfach, gleich oder verschieden substituierten Aralkylrest oder für einen Ferrocenmethylrest steht, oder

R, $R^1$ und $R^2$ gemeinsam mit dem Stickstoffatom einen Ring bilden, der durch weitere Stickstoffatome unterbrochen sein kann, und

$X^{\ominus}$ für ein Anion steht.

6. Verwendung von Jodpropargylammoniumsalzen der Formel (I) gemäss Anspruch 5 zur Bekämpfung von Schädlingen.

7. Verfahren zur Bekämpfung von Schädlingen, dadurch gekennzeichnet, dass man Jodpropargylammoniumsalze der Formel (I) gemäss Anspruch 5 auf Schädlinge und/oder ihren Lebensraum einwirken lässt.

8. Verfahren zur Herstellung von Schädlingsbekämpfungsmitteln, dadurch gekennzeichnet, dass man Jodpropargylammoniumsalze der Formel (I) gemäss Anspruch 5 mit Streckmitteln und/oder oberflächenaktiven Mitteln vermischt.

## Claims

1. Iodopropargylammonium salts of the Formula (IA)

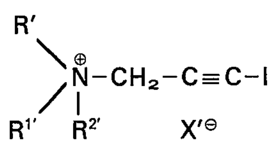
(IA)

in which

R′ and $R^{1'}$ are identical or different and represent an aliphatic radical,

$R^{2'}$ represents an aliphatic radical, an aralkyl radical which is optionally mono- or polysubstituted by identical or different substituents, or a ferrocenylmethyl radical, or R′, $R^{1'}$ and $R^{2'}$, together with the nitrogen atom, form a ring which can be interrupted by further nitrogen atoms and $X'^{\ominus}$ represents an anion,
with the exception of the compound in which R′, $R^{1'}$ and $R^{2'}$ each represent a methyl radical and $X'^{\ominus}$ represents a bromine anion.

2. Iodopropargylammonium salts of the Formula (IA) according to Claim 1, wherein

R′ and $R^{1'}$ are identical or different and represent a straight-chain or branched alkyl radical,

$R^{2'}$ represents a straight-chain or branched alkyl radical, a phenethyl or benzyl radical optionally mono- to pentasubstituted in the phenyl ring, or the ferrocenylmethyl radical, or

R′, $R^{1'}$ and $R^{2'}$, together with the nitrogen atom, form a urotropin radical and

$X'^{\ominus}$ represents a halogen, phosphate, acetate, benzoate, citrate, tartrate, sulphonate, sulphate, benzisothiazolinone or saccharin anion, with the exception of the compound in which R′, $R^{1'}$ and $R^{2'}$ each represent a methyl radical and $X'^{\ominus}$ represents a bromine anion.

3. Iodopropargylammonium salts of the Formula (IA) according to Claim 1, wherein

R′ and $R^{1'}$ are identical or different and represent straight-chain or branched alkyl with 1 to 20 carbon atoms per alkyl radical,

$R^{2'}$ represents straight-chain or branched alkyl with 4 to 20 carbon atoms, benzyl or phenethyl, it being possible in each case for the phenyl ring to be mono- to pentasubstituted by identical or different alkyl and/or halogen substituents, or ferrocenylmethyl, or

R′, $R^{1'}$ and $R^{2'}$, together with the nitrogen atom, form the urotropin radical, and

$X'^{\ominus}$ represents a chlorine, phosphate, acetate, benzoate, citrate, tartrate, benzisothiazolinone or saccharin anion, an alkyl sulphonate radical with 1 to 3 carbon atoms in the alkyl part, an aryl sulphonate radical which can optionally be mono- or disubstituted by alkyl with 1 to 3 carbon atoms or an alkyl sulphate radical with 1 to 3 carbon atoms in the alkyl radical.

4. Process for the preparation of iodopropargylammonium salts of the Formula (IA)

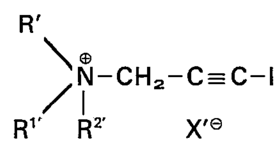
(IA)

in which

R′ and $R^{1'}$ are identical or different and represent an aliphatic radical,

$R^{2'}$ represents an aliphatic radical, an aralkyl radical which is optionally mono- or polysubstituted by identical or different substituents, or ferrocenylmethyl radical or

R′, $R^{1'}$ and $R^{2'}$, together with the nitrogen atom, form a ring which can be interrupted by further nitrogen atoms and

$X'^{\ominus}$ represents an anion, with the exception of the compound in which R′, $R^{1'}$ and $R^{2'}$ each represent a methyl radical and $X'^{\ominus}$ represents a bromine anion,
characterised in that

a) tertiary amines of the Formula (II)

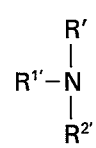
(II)

are reacted with an iodopropargyl compound of the Formula (III)

$$I-C\equiv C-CH_2-X^1 \quad (III)$$

in which

$X^1$ represents halogen or a sulphonate radical, if

appropriate in the presence of a diluent, at temperatures between 0 and 110° C, or

b) tertiary iodopropargylamines of the Formula (IV)

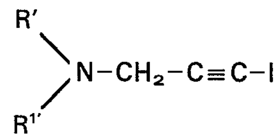

(IV)

in which

R' and $R^{1'}$ have the meanings given above, are reacted with compounds of the Formula (V)

$$R^{2'}X^{2} \qquad (V)$$

in which

$R^{2'}$ has the meaning given and

$X^{2'}$ represents halogen, alkyl sulphate or the alkyl sulphonate or aryl sulphonate radical,

if appropriate in the presence of a diluent, at temperatures between 0 and 110° C, or

c) the resulting iodopropargylammonium halides according to the invention, of the Formula (VI)

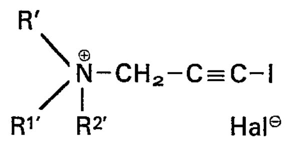

(VI)

in which

R', $R^{1'}$ and $R^{2'}$ have the meanings given above and $Hal^{\ominus}$ represents a halogen anion,

are reacted with compounds of the Formula (VII)

$$B-Y \qquad (VII)$$

in which

Y represents an anionic radical with the preferred meaning of $X^{\ominus}$, except for halogen, and

B represents an alkali metal cation or alkaline earth metal cation.

5. Pest-combating agents, characterised in that they contain at least one iodopropargylammonium salt of the Formula (I)

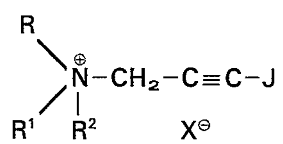

(I)

in which

R and $R^1$ are identical or different and represent an aliphatic radical,

$R^2$ represents an aliphatic radical, an aralkyl radical which is optionally mono- or polysubstituted by identical or different substituents, or a ferrocenylmethyl radical, or R, $R^1$ and $R^2$, together with the nitrogen atom, form a ring which can be interrupted by further nitrogen atoms and

$X^{\ominus}$ represents an anion.

6. Use of iodopropargylammonium salts of the Formula (I) according to Claim 5 for combating pests.

7. Method of combating pests, characterised in that iodopropargylammonium salts of the Formula (I) according to Claim 5 are allowed to act on pests and/or their habitat.

8. Process for the preparation of pest-combating agents, characterised in that iodopropargylammonium salts of the Formula (I) according to Claim 5 are mixed with extenders and/or surface-active agents.

**Revendications**

1. Sels d'iodopropargylammonium de formule (IA):

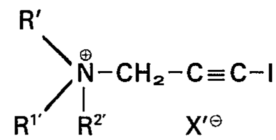

(IA)

dans laquelle

R' et $R^{1'}$ sont identiques ou différents et représentent chacun un radical aliphatique,

$R^{2'}$ représente un radical aliphatique, un radical aralkyle éventuellement substitué une ou plusieurs fois de manière identique ou différente, ou un radical ferrocène-méthyle, ou

R', $R^{1'}$ et $R^{2'}$, ensemble avec l'atome d'azote, formant un noyau qui peut être interrompu par d'autres atomes d'azote, et

$X'^{\ominus}$ représente un anion, à l'exception du composé dans lequel R', $R^{1'}$ et $R^{2'}$ représentent chacun un radical méthyle et $X'^{\ominus}$ représente un anion brome.

2. Sels d'iodopropargylammonium de formule (IA) selon la revendication 1, dans laquelle

R' et $R^{1'}$ sont identiques ou différents et représentent chacun un radical alkyle à chaîne droite ou ramifiée.

$R^{2'}$ représente un radical alkyle à chaîne droite ou ramifiée, un radical benzyle ou phénéthyle éventuellement substitué une à cinq fois dans le noyau phényle, ou le radical ferrocène-méthyle, ou

R', $R^{1'}$ et $R^{2'}$, ensemble avec l'atome d'azote, forment un radical urotropine, et

$X'^{\ominus}$ représente un anion halogène, phosphate, acétate, benzoate, citrate, tartrate, sulfonate, sulfate, benzisothiazolinone ou saccharine, à l'exception du composé dans lequel R', $R^{1'}$ et $R^{2'}$ représentent chacun un radical méthyle et $X'^{\ominus}$ représente un anion brome.

3. Sels d'iodopropargylammonium de formule (IA) selon la revendication 1, dans laquelle

R' et $R^{1'}$ sont identiques ou différents et représentent chacun un radical alkyle à chaîne droite ou ramifiée contenant 1 à 20 atomes de carbone dans chaque radical alkyle,

$R^{2'}$ représente un radical alkyle à chaîne droite ou ramifiée contenant 4 à 20 atomes de carbone, un radical benzyle ou un radical phénéthyle, le noyau phényle pouvant être chaque fois substitué une à cinq fois de manière identique ou différente par un radical alkyle et par un atome d'halogène, ou le radical ferrocène-méthyle, ou

R', $R^{1'}$ et $R^{2'}$, ensemble avec l'atome d'azote, forment le radical urotropine, et

$X'^{\ominus}$ représente un anion chlore, phosphate, acétate, benzoate, citrate, tartrate, benzisothiazolinone ou saccharine, un radical alkylsulfonate contenant 1 à 3 atomes de carbone dans la fraction alkyle, un radical arylsulfonate qui peut éventuellement être substitué une ou deux fois par un radical alkyle contenant 1 à 3 atomes de carbone, ou un radical alkylsulfate contenant 1 à 3 atomes de carbone dans le radical alkyle.

4. Procédé de préparation de sels d'iodopropargylammonium de formule (IA):

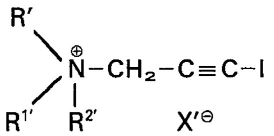

(IA)

dans laquelle

R' et R1' sont identiques ou différents et représentent chacun un radical aliphatique,

$R^{2'}$ représente un radical aliphatique, un radical aralkyle éventuellement substitué une ou plusieurs fois de manière identique ou différente, ou un radical ferrocène-méthyle, ou

R', $R^{1'}$ et $R^{2'}$, ensemble avec l'atome d'azote, forment un noyau qui peut être interrompu par d'autres atomes d'azote, et

$X'^{\ominus}$ représente un anion, à l'exception du composé dans lequel R', $R^{1'}$ et $R^{2'}$ représentent chacun un radical méthyle et $X'^{\ominus}$ représente un anion brome,

caractérisé en ce que:

a) on fait réagir des amines tertiaires de formule (III):

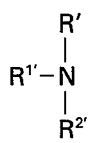

(II)

dans laquelle

R', $R^{1'}$ et $R^{2'}$ ont les significations indiquées, avec un composé iodopropargyle de formule (III):

$$I-C\equiv C-CH_2-X^1 \qquad (III)$$

dans laquelle

$X^1$ représente un atome d'halogène ou un radical sulfonate, éventuellement en présence d'un diluant, à des températures comprises entre 0 et 110° C, ou

b) on fait réagir des iodopropargylamines tertiaires de formule (IV):

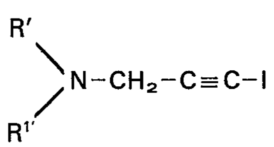

(IV)

dans laquelle

R' et $R^{1'}$ ont les significations indiquées ci-dessus, avec des composés de formule (V):

$$R^{2'}X^2 \qquad (V)$$

dans laquelle

$R^{2'}$ a la signification indiquée ci-dessus, et

$X^2$ représente un atome d'halogène, un radical alkylsulfate ou un radical alkyl- ou arylsulfonate, éventuellement en présence d'un diluant à des températures comprises entre 0 et 110° C, ou

c) on fait réagir les halogénures d'iodopropargylammonium de formule (VI) obtenus suivant l'invention:

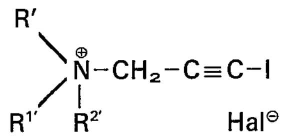

(VI)

dans laquelle

R', $R^{1'}$ et $R^{2'}$ ont les significations indiquées ci-dessus, et

$Hal^{\ominus}$ représente un anion halogène,

avec des composés de formule (VII):

$$B-Y \qquad (VII)$$

dans laquelle

Y représente un radical anionique avec la signification préférée de $X^{\ominus}$, excepté un atome d'halogène, et

B représente un cation alcalin ou alcalino-terreux.

5. Parasiticides, caractérisés en ce qu'ils contiennent au moins un sel d'iodopropargylammonium de formule (I):

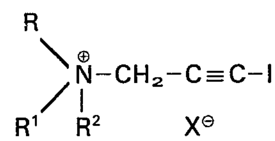

(I)

dans laquelle

R et $R^1$ sont identiques ou différents et représentent chacun un radical aliphatique,

$R^2$ représente un radical aliphatique, un radical aralkyle éventuellement substitué une ou plusieurs fois de manière identique ou différente, ou un radical ferrocène-méthyle, ou

R, $R^1$ et $R^2$, ensemble avec l'atome d'azote, forment un noyau qui peut être interrompu par d'autres atomes d'azote, et

$X^{\ominus}$ représente un anion.

6. Utilisation de sels d'iodopropargylammonium de formule (I) selon la revendication 5, pour combattre les parasites.

7. Procédé en vue de combattre les parasites, caractérisé en ce qu'on fait agir des sels d'iodopropargylammonium de formule (I) selon la revendication 5, sur les parasites et/ou leur biotope.

8. Procédé de préparation de parasiticides, caractérisé en ce qu'on mélange des sels d'iodopropargylammonium de formule (I) selon la revendication 5, avec des diluants et/ou des agents tensio-actifs.